Europäisches Patentamt

European Patent Office

Office européen des brevets

(19)

(11) Publication number: **0 144 774**
**A2**

(12) **EUROPEAN PATENT APPLICATION**

(21) Application number: **84113411.7**

(22) Date of filing: **07.11.84**

(51) Int. Cl.⁴: **H 04 R 25/00**
**A 61 F 11/04**

(30) Priority: **07.11.83 IL 70153**

(43) Date of publication of application:
**19.06.85 Bulletin 85/25**

(84) Designated Contracting States:
**AT BE CH DE FR GB IT LI LU NL SE**

(71) Applicant: **Navot Technology Ltd.**
**11 Chibat-Zion Street**
**Ramat Gan(IL)**

(72) Inventor: **Kanevsky, Dimitry**
**Gilo 303/54**
**Jerusalem(IS)**

(72) Inventor: **Ophir, Joseph**
**36 Hamalot St.**
**Givataim(IS)**

(72) Inventor: **Leibman, Vadim**
**5a Vradim St.**
**Rekhasim(IS)**

(72) Inventor: **Skurkovich, Gregory**
**10 Ben Tabbai St.**
**Jerusalem(IS)**

(74) Representative: **KUHNEN & WACKER**
**Patentanwaltsbüro**
**Schneggstrasse 3-5 Postfach 1729**
**D-8050 Freising(DE)**

(54) **Audiotactile communication system.**

(57) An audiotactile communication system including input apparatus including apparatus for converting audio signals into input electrical signals related to the audio signals, circuit apparatus responsive to the electrical signals for producing first or second output electrical signals, the first signals being produced when a DC signal which is a function of the input electrical signals is not less than a selected reference DC signal and the second signals being produced when the DC signal is less than the selected reference DC signal, and transducer apparatus responsive to the output electrical signals for providing tactile signals adapted to be sensed by a person.

EP 0 144 774 A2

./...

Croydon Printing Company Ltd.

FIG. 1

.0144774

02817 diskette 33 5.10.84

## AUDIOTACTILE COMMUNICATION SYSTEM

The present invention generally relates to hearing devices and, more particularly, to a system for converting audio signals into vibrations or other sensible indications communicated to a deaf or hard of hearing person.

Many aids for deaf or hard of hearing persons have been developed over the years. These aids include the known hearing aids typically worn by a hard of hearing person in or near the ear. The disadvantages of these devices are well known.

Briefly, they can only aid people whose hearing nerves are not totally damaged. They cannot help totally or nearly deaf people, since all they do is amplify audio signals, over the entire spectrum or particularly chosen ranges. Even those that theoretically can be helped by such devices, find them troublesome and a nuisance, since they also amplify ambient noise, which is most disturbing.

As to aiding the deaf person for whom a hearing aid is useless, since his hearing nerves are medically non-responsive to audio signals, other devices have been proposed. In some of these devices, speech, i.e. audio signals, are converted to types of signals which are applicable to bones and/or nerves and by so doing are intended to communicate speech to the deaf by other than the hearing nerves. The devices described in U.S. Patent Nos. 1,733,605, 2,150,364 and 4,139,742 are examples of some such devices. All of the devices known to applicant have

0144774

limited practical use, if any, for one or more of the following reasons: They are not sufficiently selective to transmit the necessary information in the sensitivity spectrum of cutaneous nerves, they are not designed to capture all meaningful audio information, they are affected by noise and they are too complex for practical use.

A need therefore exists for a reliable, yet relatively simple device for communicating audio signals to a very hard of hearing or totally deaf person by converting the audio signals into signals which can be applied through a person's skin to nerves or the like, which the person can sense and learn to interpret as audio signals. Such a device is effectively an audio tactile communication device.

· The present invention is directed to provide such a device. The device of the present invention may be defined as an audiotactile communication system comprising:

input apparatus including apparatus for converting audio signals into input electrical signals related to the audio signals;

circuit apparatus responsive to the electrical signals for producing first or second output electrical signals, the first signals being produced when a DC signal which is a function of the input electrical signals is not less than a selected reference DC signal and the second signals being produced when the DC signal is less than the selected reference DC signal; and

transducer apparatus responsive to the output electrical signals for providing tactile signals adapted to be sensed by a person.

Further in accordance with an embodiment of the present invention the first signals are at a first frequency and are of an amplitude related to the DC signal and the second signals are at a second frequency and are of of an amplitude related to the input electrical signal.

Additionally in accordance with an embodiment of the present invention, the transducer apparatus includes first and second transducers providing tactile signals at respective first and second locations on a person. Alternatively, only a single transducer may be employed.

Further in accordance with an embodiment of the present invention there is also provided portable apparatus for real-time speech element identification comprising:

input apparatus for receiving an input electrical signal which may contain speech;

variable delay line apparatus coupled to said input apparatus for receiving the input electrical signal;

apparatus for selectively governing the input and output clocking rate to the variable delay line apparatus so as to vary the effective output frequency range of the output thereof, thereby scanning a relatively wide frequency spectrum of the incoming electrical signal at a relatively high speed and providing a delayed output signal of a relatively narrow frequency bandwith;

filter apparatus for receiving the delayed output signal and having a bandpass corresponding to the frequency range of the delayed output signal; and

detector apparatus receiving the output of the filter apparatus for determining the presence of speech elements having characteristic frequency spectra.

It is also a particular feature of the present invention that programmable automatic gain control circuitry providing stabilized gain in the presence of speech from a single source is provided.

The novel features of the invention are set forth with particularity in the appended claims. The invention will best be understood from the following description when read in conjunction with the accompanying drawings.

Figure 1 is a general block diagram of one embodiment of the invention;

Figures 2a-2f are waveform diagrams useful in explaining the embodiment of Fig. 1;

Figure 3 is essentially a block-schematic diagram of part of the circuitry shown in Fig. 1;

Figure 4 is a diagram of yet another embodiment of the invention;

Figure 5 is a block diagram of a real-time character identification arrangement; and

Figure 6 is a block diagram illustration of a preferred embodiment of control unit employed in the apparatus of Fig. 5.

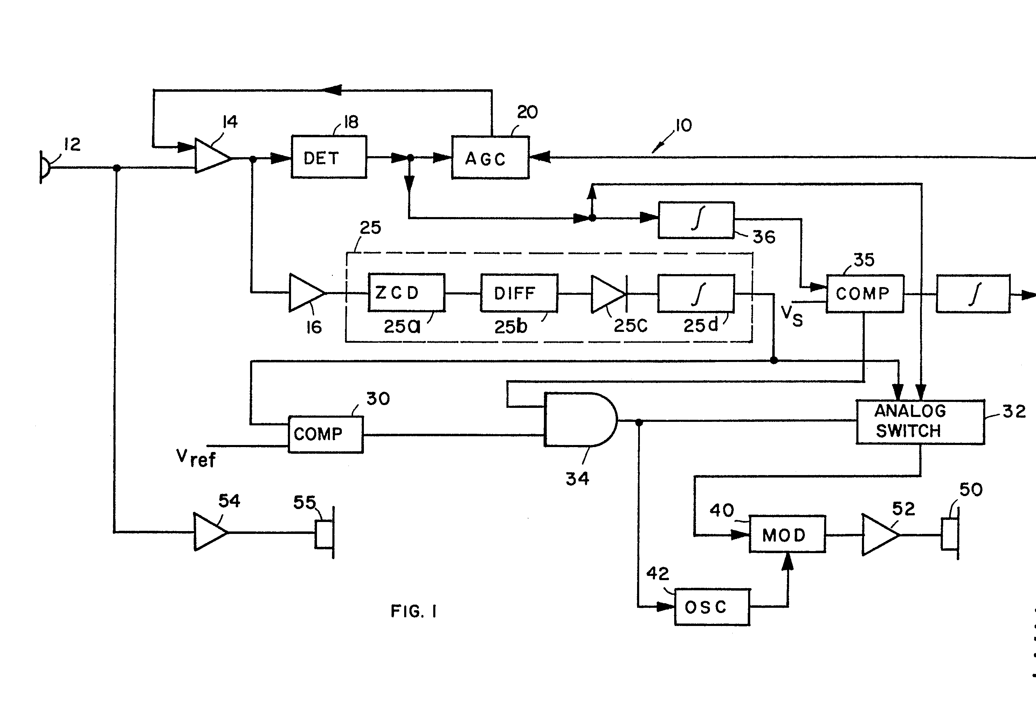

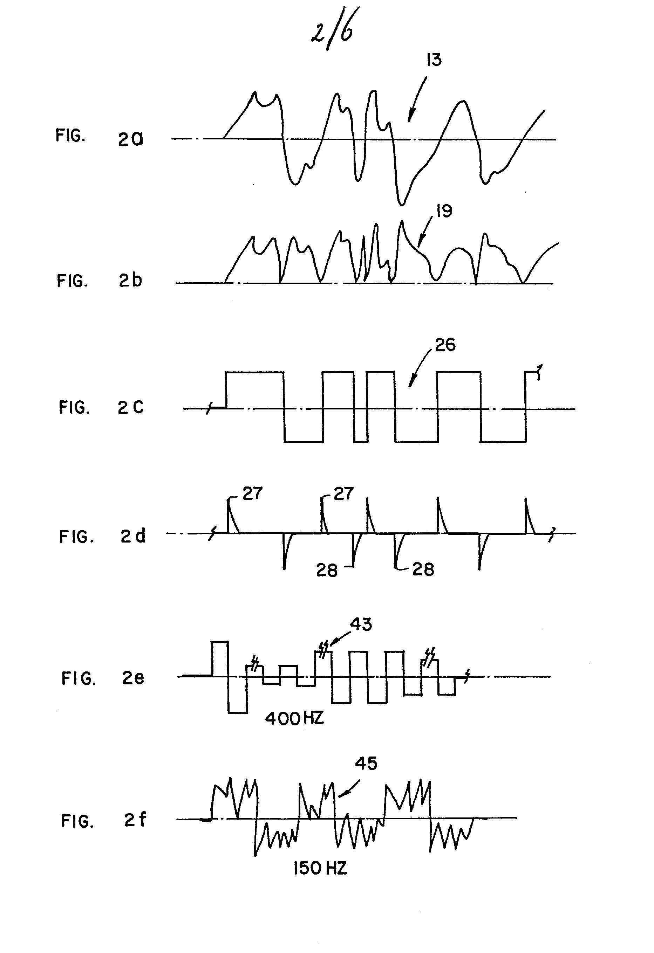

Attention is now first directed to Fig. 1 which is a block diagram of one embodiment of the invention and to Figs. 2a - 2f in which waveforms are diagrammed. In Fig. 1 the novel audiotactile device of the invention or simply the device is designated by 10. It is shown including a microphone 12 which converts audio signals, i.e. speech, to electrical signals as shown in Fig. 2a, and designated therein by 13. The output of microphone 12 is supplied to an amplifer 14 which is connected to another amplifier 16 and to an absolute value detector 18. Thus, its output is a D.C. variable amplitude waveform as shown in Fig. 2b and designated by 19. This D.C. output is applied to automatic gain control circuitry or simply AGC 20, which controls the gain of amplifier 14 as will be described hereafter. The automatic gain control circuitry of the present invention is of particular importance since it comprises programmable apparatus which is operative to maintain a stabilized gain level in the presence of detected speech from a single source. Thus the potentially disturbing phenomenon of gain changes in the course of speech is obviated.

The output of amplifier 16 which is the amplified A.C. waveform from amplifier 14 is applied to a speech detector 25. Assuming the output of amplifier 16 to be an amplified format of waveform 13 in Fig. 2a, the speech detector includes a zero crossing detector 25a whose output is as shown in Fig. 2c and is designated by 26.

The output of the zero crossing detector is supplied to a differentiator 25b which produces both positive and negative pulses designated 27 and 28 as shown in fig. 2d. The pulses are passed through a diode 25c thus clipping the negative pulses 28, to an integrator 25d whose output is a D.C. voltage whose amplitude is a function of the number of positive pulses 27 per unit time.

The output of integrator 25d, which represents the output of the speech detector 25, is thus a D.C. voltage which is a function of the frequency of the incoming electrical signals 13. This output is applied as one input to a comparator 30, and as one input to an analog switch 32. The other input to comparator 30 is a selected reference D.C. voltage designated Vref, while another input to the analog switch 32 is the output of detector 18, represented by waveform 19 in Fig. 2b. Whenever the output of speech detector 25 is not less than Vref the output of comparator 30 to an AND gate 34 is high. The other input to gate 34 is the output of a comparator 35 which compares the output of an integrator 36 with a reference voltage $V_s$, representing speech.

Comparator 35 provides a high output to gate 34 only when the output of integrator 36, which integrates the output of detector 18 is higher than $V_s$. Thus gate 34 is enabled only when the outputs of comparators 30 and 35 are both high, the latter's output being high only when speech is assumed to have been detected. Therefore, gate 34 is enabled only when the output of integrator 36 is high.

When gate 34 is enabled it provides a high output to analog switch 32 which switches to a state in which the output of speech detector 25 is passed through it to a modulator 40. Also, the

0144774

output of AND gate 34 is supplied as a control signal to a two-frequency oscillator 42, the two frequencies being assumed to be 400 Hz and 150 Hz, which are supplied to modulator 40 when the output of gate 34 is high or low, respectively. Thus the output of switch 32 modulates the frequency supplied to the modulator.

From the foregoing it should be apparent that, when speech is present, as indicated by a high output from comparator 35, and the D.C. output of speech detector 25 is not less than Vref, switch 32 passes the D.C. output of speech detector 25 to the modulator. The latter, receiving 400 Hz from oscillator 42 modulates it by the D.C. output from detector 25 to provide a square wave signal at 400 Hz with an amplitude dependent on the amplitude of the D.C. output from speech detector 25. Such an output is shown in Fig. 2e and designated by 43. Alternatively, instead of a square wave signal, a sinusoidal signal or signal of any other suitable configuration may be employed. The use of a sinusoidal signal has the advantage that it does not contain harmonics which result in acoustical feedback.

On the other hand, if the amplitude of the D.C. output of speech detector 25 is less than Vref the output of comparator 30 is low and thus gate 34 is disabled. Consequently, analog switch 32 passes the output of detector 18 (shown in Fig. 2b) to the modulator, which is also supplied with 150 Hz. Thus the output of the modulator is as shown in Fig. 2f designated by 45. The output of modulator 40 is fed to a tactile transducer 50 after amplification by an amplifier 52. In addition, the output of microphone 12 is supplied directly to an amplifier 54 whose output is supplied to another tactile transducer 55. The tactile transducer typically comprises a vibration generator such as an electromagnetic bone conductor or a piezoelectric vibrator, both of which are commercially available. Alternatively the tactile transducer may comprise an electrode or any other suitable transducer.

It is appreciated that under certain circumstances the transducer 55 may be eliminated and the output of amplifier 54 may be transmitted via transducer 50.

As is appreciated, audio signals cover a wide range of frequencies, up to 20,000 Hz (20 kHz). Speech is confined to the lower portion of the audio range, typically up to several thousand Hz, e.g. 6000 Hz. The nerves of the skin have a much lower frequency response. It is on the order of not more than about 500 Hz. Thus, the signals which tactile electrode 50 applies to the nerves through the skin should have a carrier frequency within this band.

There are several sounds such as the "sh" in shoot or the "s" in see or the "ch" as in chalk, which have natural frequencies in the range of 2000 Hz to 7000 Hz. The identification of such sounds by a deaf person is of paramount importance. This is achieved in the present invention by the incorporation of the speech detector 25 and the circuitry controlled by its output. The Vref is chosen so that when any of these hard to detect sounds are received by microphone 12, the D.C. output of speech detector 25 is not less than Vref. Thus, the person receives, via electrode 50, 400 Hz square wave pulses whose amplitudes change as a function of the frequency of these sounds. By sensing the 400 Hz pulses and their intensities, i.e. amplitudes, the person learns not only to recognize that such sounds are present but also which of these is communicated to him.

As to the other sounds, the person receives square wave pulses at 150 Hz which are modulated by the other sounds. After a reasonably short training period the person learns to distinguish between them based on the modulation of the 150 Hz square wave pulses, as shown by waveform 45 in Fig. 2f. This is due to the fact that various sounds have characteristic waveforms which a person can learn to identify.

As to the output of tactile electrode 55, its frequency waveform is that of regular speech. It is too high for the nerves to distinguish individual frequencies. However, by providing it to the person at nerves other than the nerves at which electrode 50 is applied, the nerves to which the pulses from electrode 55 are applied sense the waveform. Thus, while individual sounds cannot be made out, the presence of speech as well as its level or amplitude and interruptions therein are communicated to the person. This information combined with the signals from electrode 50 enables the person to decipher the sounds communicated to him, thus enabling him to hear through the signals sent by the stimulated nerves to the brain.

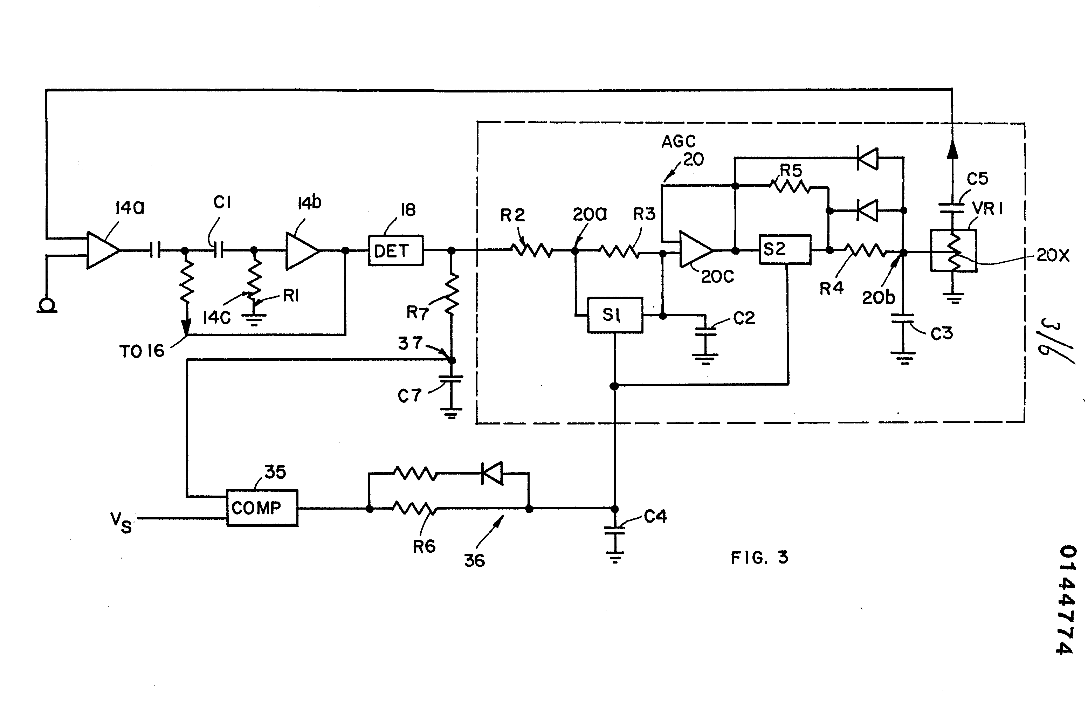

Before proceeding to describe other features of the invention, attention is directed to Fig. 3 in connection with which one embodiment of the AGC unit 20 will be described in further detail. Briefly, amplifier 14 is a two-stage amplifier represented by 14a and 14b with a high pass filter formed of

capacitor C1 and resistor R1.    This filter is designed to filter out low frequency noise.    The output of amplifier 14b is fed to detector 18 whose output is fed to comparator 35 via integrator 37, comprising resistor 7 and capacitor C7, and to AGC 20.

The latter consists of two integrators, integrators 20a and 20b with different and variable time constants and an amplifier 20c.    Integrator 20a consists of a capacitor C2 and two resistors· R2 and R3, the latter shunted by an analog switch S1.    Originally S1 is closed thus shorting R3.    When S1 is open, R3 is in series with R2.    Thus, the time constant of integrator 20a is variable between $R2C2$ and $(R2+R3)C2$.    Likewise, integrator 20b is made up of a capacitor C3 and resistors R4 and R5, the latter shunted by analog switch S2, which acts like switch S1.    Thus the time constant of integrator 20b is variable between $R4C3$ and $(R4+R5)C3$.    The output of integrator 20b controls a voltage controlled variable resistor VR1 so that the level at point 20x, which is fed back to amplifier 14a via a capacitor C5, is a function of the gain control.    The states of the two analog switches S1 and S2 are controlled by the output of integrator 36 which includes a capacitor C4 and a resistor R6 which is fed by comparator 35.

In operation the time constant of integrator 37 and that of integrator 20a when R3 is shorted out by switch S1 is very short on the order of a few ms., e.g. 4 ms. in order not to lose any speech once present, while preventing noise from appearing as speech.    The time constant of integrator 20b with R5 shunted is about 400 ms.

If audio signals pass filter 14c, comprising resistors R1 and C1, for over 4 ms. the input to comparator 35 for integrator 36 is higher than $V_s$. Thus the output of comparator 35 to integrator 36 is high. Integrator 36 integrates the output for a few hundred milliseconds, e.g. 200 ms and opens switches S1 and S2. Thus the time constants of 20a and 20b change. In one embodiment they change from 4 ms and 400 ms to 40 ms and 4 seconds respectively.

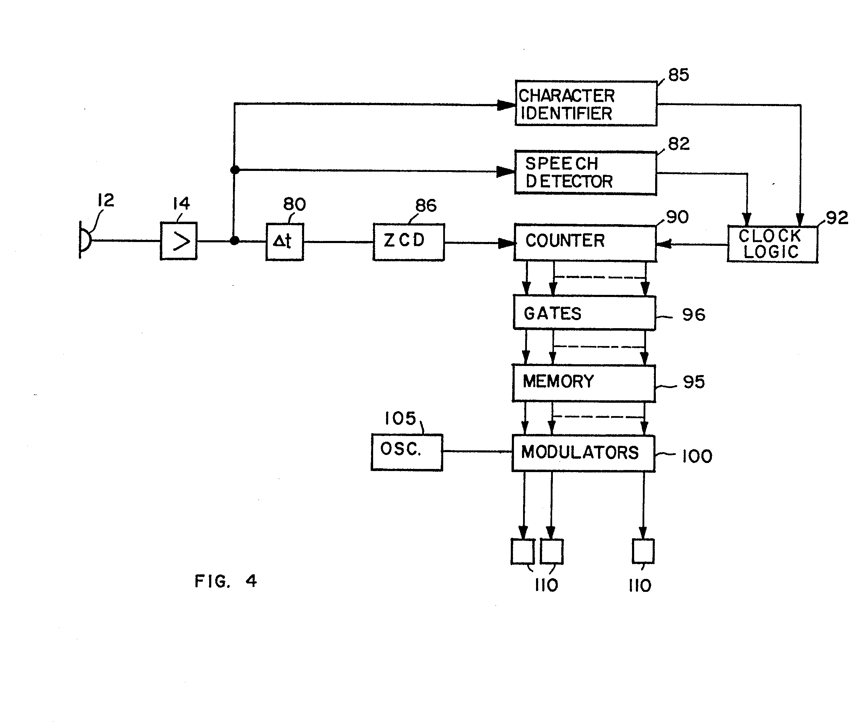

In the foregoing the audiotactile system has been described in an analog embodiment. However, the invention is not intended to be limited thereto. Attention is now directed to Fig. 4 in connection with which a digitally implemented embodiment will be described.

Briefly, the output of microphone 12 which converts input audio signals into related input electrical signals is amplified by amplifier 14. The output of amplifier 14 is supplied to several devices. These include a delay unit 80, a speech detector 82 and a character identifier 85. The output of delay unit 80 is fed to a pulse former such as a zero crossing detector 86 which is connected to a clockable counter 90. The outputs of detector 82 and identifier 85 are fed to a logic controller 92. Briefly the speech detector 82 acts as a control gate. It, as previously explained, distinguishes noise from speech. When speech is present it enables controller 92 to respond to the control signals from character identifier 85. The latter, by means of control signals, based on the character which it identifies, causes controller 92 to clock the counter 90 during chosen instances and durations and thereby cause the counter 90 to receive pulses from detector 86, which pulses are produced via amplifier 14 and delay unit 80 from the input electrical signals.

As is known by those familiar with the art, different characters or letters of speech have not only different frequencies but also different characteristic waveforms or frequency patterns.

For example, letters like "A", "K", "J", have relatively low frequency patterns as compared to letters such as "C", "P", "Z", which also differ from letters such as "L", "M", "N" and the like, whose frequency pattern is concentrated in the lower part of the speech spectrum. This phenomenon is well known and has been discussed in the literature. For example, in a book entitled "Acoustic Theory and Synthesis of Speech with Application to Swedish" by G. Faut, published in Stockholm, Sweden in 1959, spectra in terms of frequencies and relative amplitudes for different sounds are diagrammed and discussed.

Based on known spectra of characters, the character identifier 85 is designed to identify each received character by its frequency and pattern as received from amplifier 14. Once the character is identified, based on prestored information for each possible received character or sound, identifier 85 sends control signals to controller 92, which are operative when speech is present as indicated by speech detector 82, to define a character period during which the counter counts. The counter, when so clocked, receives delayed pulses, in digital form after the delay interposed by delay 80. The number of these pulses counted by counter 90 corresponds to the character which identifier 85 has identified.

At the end of a character period, a number of pulses are present in counter 90 at different stages thereof, which number is characteristic of an identified character. At the end of a character period, the contents of the stages of counter 90 as binary 1's or 0's are clamped in parallel into a memory 95 through gates 96 which are opened for a brief period on the order of micro- or nano- seconds.

Thereafter, as a subsequent character is being received and identified, the pulses indicative of the previously identified characters are stored in memory 95. The latter can be thought of as a multistage buffer, e.g. five stages with parallel input and parallel output.

The system further includes a plurality of modulators 100, each receiving a signal from an oscillator 105, and supplying a driving signal to a different tactile transducer 110. For explanatory purposes, each modulator may be assumed to be a gate which feeds the frequency of oscillator 105 to its associated transducer 110, when the input to the modulator is of one binary value, e.g. a "1", and blocks the supply of the oscillator output to the transducer when the memory output is an "0". Thus, for each received character a combination of presence or absence of tactile signals in the transducers 110, assumed to be located at different locations on the body, is provided. A person can easily learn to "recognize" a character by its unique combination of tactile signals received via the plurality of transducers 110. Clearly, if desired, a plurality of oscillators providing different frequencies to the different oscillators may be used. Also the 1's and 0's at each output of memory 95 may be used to control the amplitude of the signal supplied to each transducer rather than control the presence or absence of a signal thereat.

As previously stated and as is appreciated by those familiar with the art, spoken characters have unique characteristic frequency patterns at different frequencies or bands thereof.. The operation of the embodiment just described in connection with Fig. 4 is dependent of the character identifier 85. The latter, receiving the speech from amplifier 14 (Fig. 4), analyzes it to identify which character is received. Based on such analysis, identifier 85 causes controller 92 to define the character periods during which pulses from zero crossing detector 86 are clocked into the counter 90.

In order to identify a character in essentially real time the identifier 85 can be implemented with known character identification devices. As is appreciated by those familiar with the art, all such devices employ a large number of circuits which operate in parallel, each circuit having its own filtering circuitry or filters. Thus, in the prior art a large number of filters in separate parallel operated circuits are required. One such prior art system is described in U.S. Patent No. 4,100,370 entitled "Voice Verification System Based on Word Pronounciation", the disclosure of which is incorporated herein by reference. It is clear therefrom and from other known multifilter devices that such a system, though implementable, is quite complex and therefore expensive and not portable. As will become apparent from the following discussion, in accordance with the present invention a system for character identification in real time is provided in which prior art disadvantages are eliminated.

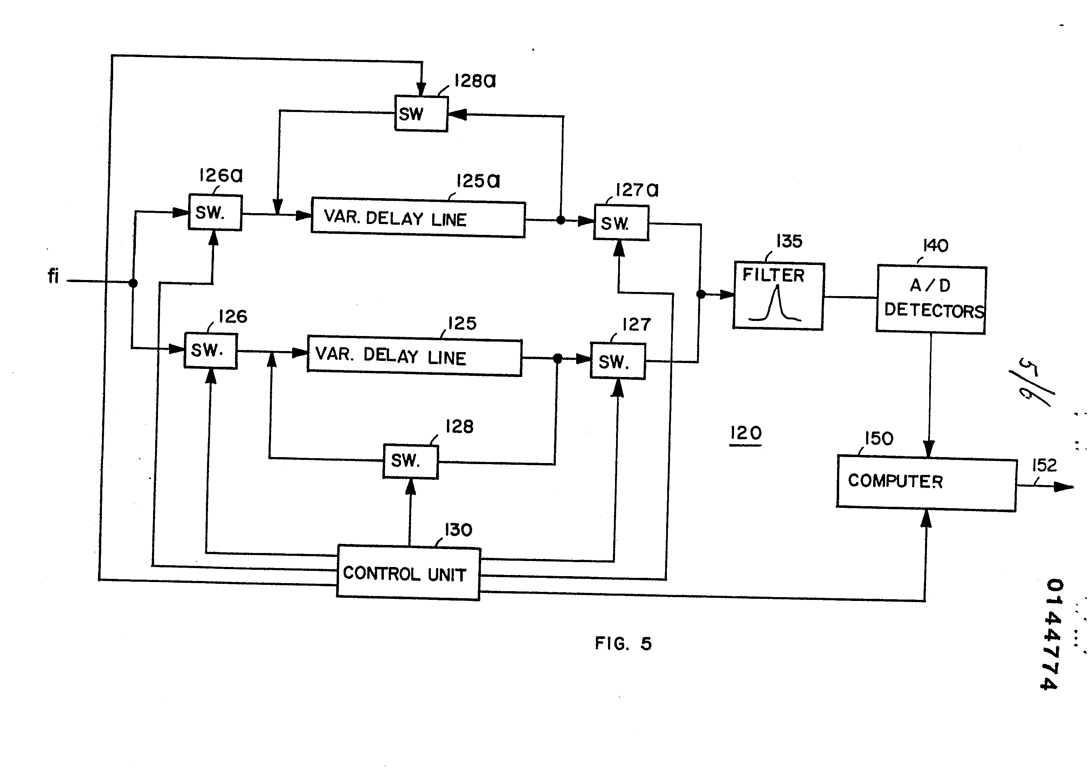

The character identification system or character identifier, shown in block form in Fig. 5 and designated by 120 will be described as implementable by analog and digital circuitry. However, as will become apparent, all digital means may be employed. The character identifier 120 receives input signals, representing incoming signals having an unknown frequency or unknown frequencies designated by $f_i$ and different amplitudes. Based on the known frequencies and their relative amplitudes, i.e. the spectra of known characters, identifier 120 identifies the incoming spectra and thus identifies the character. Such identification is done essentially in real time.

Identifier 120 includes a variable delay line 125 which is, at first, assumed to be analog. The incoming signals having frequencies $f_i$ are supplied thereto through an analog gate or switch 126 which, together with other circuits, is under the control of a control unit 130. The output of the delay line 125 is fed through a switch 127 to a filter 135. The output and input of delay line 125 are interconnected through a switch 128, which is also controlled by unit 130.

Let it be assumed that the incoming frequency $f_i$ is 100 Hz, designated $f_{100}$. If $f_{100}$ is clocked into delay line 125 at a certain clocking-in frequency and clocked out at the same frequency, then the frequency of the clocked out signals appears as 100 Hz. However, if the clocking out ($f_{out}$) frequency is

0144774

greater or smaller than the clocking-in ($f_{in}$) frequency, the frequency of the clocked out signals is greater or smaller than the frequency of clocked-in signals, by the ratio of the clocked-in frequency to the clocked-out frequency. For example, if $f_{out}$ is twice $f_{in}$, i.e. $f_{out}/f_{in} = 2$, then the frequency of the clocked out signals, designated $f_o$ is $f_i \times (f_{out}/f_{in})$. In the particular example, if $f_i = 100$ Hz, and $f_{out}/f_{in} = 2$, then $f_o =$ 100 x 2 = 200 Hz.

Thus, the frequency of the incoming signals $f_i$, which can be fed to filter 135 can be varied by the clocking in and clocking out of the signals into and out of delay line 125. That signals clocked in at one frequency appear to be of a higher frequency if clocked out at a higher frequency is clear when considering the fact that music on a phonograph record or tape appears to be of a higher frequency when played back at a higher speed than required. By controlling switch 128 the clocked out signals can be recirculated into the delay line 125 and clocked again over and over. Preferably the amplification of delay line 125 is unity.

Filter 135 is a bandpass filter. Its output is supplied to a group of detectors 140, with A/D converters which are designed to sense the amplitudes of signals at a plurality of different discrete frequencies within the band of filter 135. Thus, by varying the ratio of clocked-in frequency, one can determine the frequency or frequencies of the incoming signals, based on the output of detectors 140. Since each speech character has a unique signal spectrum in terms of frequencies and amplitudes, by the arrangemnt shown in Fig. 5 one can identify each character by comparing the outputs of detectors 140, which together are indicative of characteristic portions of the spectrum of the incoming signals $f_i$, with prerecorded spectra, or characteristic portions thereof, of all possible characters to be identified.

Present day delay lines are clockable at very high frequencies. Therefore one can clock in incoming signals and repeatedly sample and reclock them at different frequencies. Each sampling fed to filter 135 and therefrom to detectors 140 is effectively a different frequency component of the spectrum of the unknown character, but yet within the band of filter 135. Thus, with the arrangement shown in Fig. 5 a single filter 135 can be used to analyze the frequency spectrum of the character to be recognised. It should also be appreciated that by using a higher clocking out frequency, the bandpass is significantly greater and therefore information retrieval time is greatly reduced. This is important, particularly when the variable delay line and the single filter are used to analyze a spectrum of information other than speech, e.g. in an information communication system, radar or the like.

While one character is identified, signals, representing a following character in the speech train, may be fed to another delay line interconnected with three switches such as 126-128. The second delay line and switches are designated by 125a and 126a, 127a and 128a.

It is thus seen that with a single filter 135 and recirculating delay line 125 controlled at different clocking-in and clocking-out frequencies, one can identify in real time a plurality of discrete frequencies extending over the entire frequency spectrum of the character to be identified. In the prior art a large number of filters, one for each discrete frequency of interest are required for such identification. Thus, the arrangement of Fig. 5 described so far can be produced as · a very small and relatively inexpensive device for spectrum analysis. Although it is described herein for the purpose of· identifying character frequency spectra, it can clearly be used to analyse frequency spectra other than those of speech.

If desired, the time for each character analysis can be shortened by adding only one more filter to filter 135. By tuning the two filters to significantly different center frequencies, the entire audio range of interest can be analyzed with half as many recirculations. Thus, although in Fig. 5, a ·single filter 135 is shown, it should be regarded as representing one or several, e.g. 2 or 3 filters.

As previously stated, the output of filter 135 or filters, if several are employed, are fed to the detectors 140 which include A/D converters, after each recirculation of the delay line. Thus, they store the amplitude of the output of filter 135 after each recirculation, each output representing the amplitude of a particular frequency component of the input signals. The contents of the detectors are thus indicative of the amplitudes and frequencies of the components of the character. They are compared with prestored amplitude and frequency components of each of a plurality of known characters in a device 150. Device 150 can be a computer or the like, which is also controlled by control unit 130. When the detectors 140 contain spectral information which matches that stored in device 150 for a particular character, the character is identified by appropriate signals on output lines 152.

In the embodiment shown in Fig. 4, such signals may be supplied to controller 92 to control the clocking in of the pulses into counter 90. If desired, since the signals on lines 152 are indicative of the character which was identified, one can use these signals directly as a parallel set of 1's and 0's to modulate a signal of a frequency, sufficiently low to be sensed by the skin, and fed to a corresponding set of different modulators which are, in turn, connected to a set of different transducers, such as transducers 110, shown in Fig. 4.

To complete the description of Fig. 5, as previously pointed out, the device 150 serves to store spectral data of known characters with which the data in detectors 140 is compared to identify the character which is most likely to have produced the data or spectrum represented by the contents of detectors 140. Device 150 can be programmed to perform the necessary comparisons to identify the character as herebefore explained.

As to the additional delay line 125a and additional switches 126a, 127a and 128a, the delay line 126a is used to store speech-produced incoming signals while those previously received and entered in delay line 125 are recirculated until the character is identified. Then, unit 130 controls the switches to circulate the signals in delay line 125a until the character represented by them is recognized, while delay line 125 is used as a temporary buffer for a subsequent character. Thus, with two delay lines and their associated switches, none of the audio signals are lost. Clearly, the circuitry, shown in Fig. 5, can be implemented by all analog, all digital or analog-digital circuits.

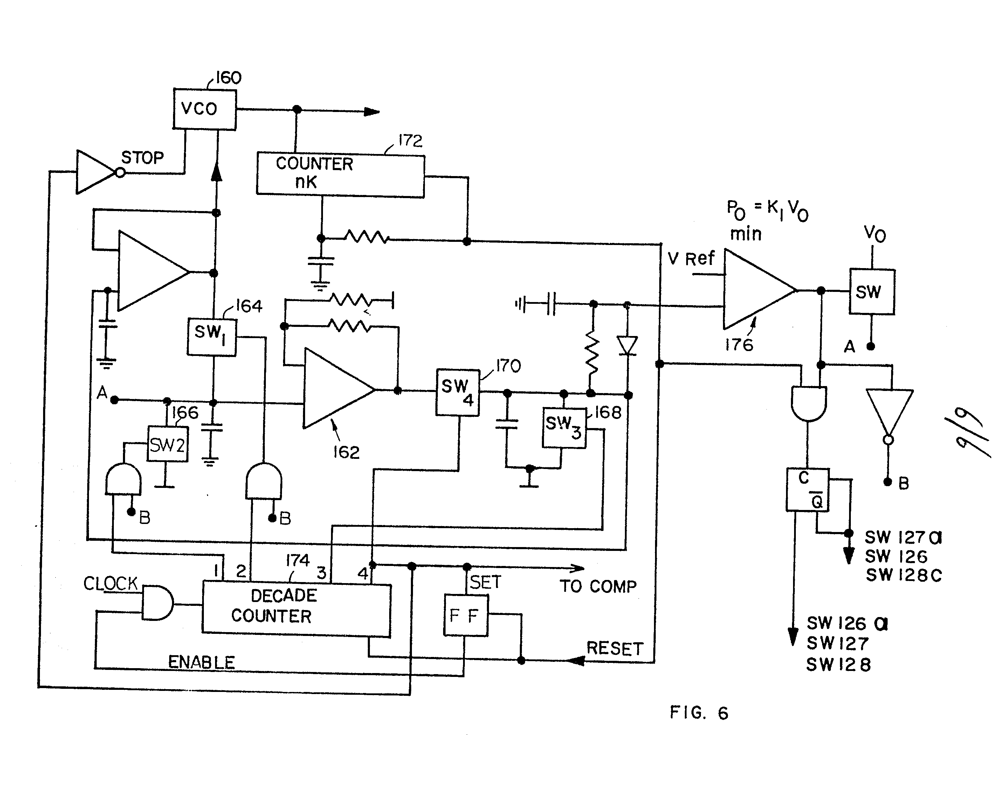

Reference is now made to Fig. 6 which illustrates a preferred embodiment of control unit 130. A linear voltage controlled oscillator 160, such as an 14046 of Motorola, is operative when an initial reference voltage $V_o$ is applied to a point A, to receive an initial reference voltage $V_o$ multipled by a coefficient determined by an operational amplifier.

During operation of the control unit, a four stage amplification loop comprising switches 164, 166, 168 and 170 is operative to increase the voltage supplied to voltage controlled oscillator 160 in predetermined steps over a predetermined range over a predetermined time, thus causing oscillator 160 to provide a stepwise scanned frequency output.

Counters 172 and 174 keeps track of recirculation of information through the delay lines and frequency scanning therethrough with the help of a comparator 176 thus enabling controller 130 to provide correct signals to switches 126 - 128.

Although particular embodiments of the invention have been described and illustrated herein, it is recognized that modifications and variations may readily occur to those skilled in the art and consequently, it is intended that the claims be interpreted to cover such modifications and equivalents.

- 1 -

CLAIMS

0144774

1. An audiotactile communication system comprising:

input means including means for converting audio signals into input electrical signals related to the audio signals;

circuit means responsive to said electrical signals for producing first or second output electrical signals, said first signals being produced when a DC signal which is a function of the input electrical signals is not less than a selected reference DC signal and said second signals being produced when said DC signal is less than said selected reference DC signal; and

transducer means responsive to said output electrical signals for providing tactile signals adapted to be sensed by a person.

2. An audio tactile communication system according to claim 1 and wherein said first signals are at a first frequency and are of an amplitude related to said DC signal and said second signals are at a second frequency and are of an amplitude related to said input electrical signal.

3. An audiotactile communication system according to either of the preceding claims and wherein said transducer means includes first and second transducer means providing tactile signals at respective first and second locations on a person.

0144774

4.      An audiotactile communication system according to  any of  the preceding claims and wherein said D.C.  signal is related to the frequency of said electrical signals.

5.      An audiotactile communication system according to claim 4 wherein  each  of said first and  second  frequencies  is  not greater than 500 Hz.

6.      An audiotactile communication system according to claim 1  wherein  said  circuit  means  include  detection  means  for converting the input electrical signals into unipolar signals and means  responsive  to  said  unipolar  signals  for  modulating therewith  signals  at said second frequency for  producing  said second output electrical signals.

7.      An audiotactile communication system according to claim 6 wherein said circuit means for generating the D.C.  signal as a function  of  the  input  electrical signals  include  means  for sensing the zero crossings of said input electrical signals,  for generating  impulse  pulses related thereto and  for  integrating said pulses to provide said D.C. signal.

8.      An audiotactile communciation system according to claim 7 wherein said selected reference D.C.  signal is indicative  of audio signals above a selected frequency,  which is not less than n Hertz (Hz), wherein n is not less than 1,000.

- 3 -

0144774

9.      An audiotactile communication system according to claim 8 wherein n is not less than 2,000.

10.     An audiotactile communication system according to claim 9 wherein said input means include amplification means for amplifying the input electrical signals and gain control means for controlling the amplification provided by said amplification means, said gain control means being integration means with first and second variable time constants.

11.     An audiotactile communication system comprising:

input means responsive to audible speech for providing related input electrical signals;

means for storing information related to known speech components;

means for comparing at least a portion of the input electrical signals with said stored information to identify the speech represented by said portion; and

output means including digital-signal-producing means responsive to the identified speech for producing a plurality of signals within the tactile sensitivity of a person and for applying said plurality of signals to a person.

12.     An audiotactile communication system according to claim 11 wherein said output means include means for controlling the modulation of signals at a preselected frequency within the tactile sensitivity of a person with digital signals produced in said output means.

13.     An audiotactile communication system according to claim 12 wherein said output means includes a plurality of tactile transducers attachable at selected locations on a person's body, and means for applying the modulated signals controlled by said digital signals to said transducers.

14.     An audiotactile communication system according to claim 11 wherein said system includes clockable variable delay line means and control means for repeatedly clocking a portion of said input electrical signals into and out of said delay line means at selected different frequencies and for applying each set of clocked out signals to filter means of a preselected bandpass.

15.     Apparatus for real-time speech element identification comprising:

input means for receiving an input electrical signal which may contain speech;

variable delay line means coupled to said input means for receiving said input electrical signal;

means for selectively governing the input and output clocking rates of said variable delay line means so as to vary the effective output frequency range of the output thereof, thereby scanning a relatively wide frequency spectrum of the incoming electrical signal at a relatively high speed and providing a delayed output signal;

filter means for receiving the delayed output signal 0144774 and having a bandpass corresponding to the frequency range of the delayed output signal; and

detector means receiving the output of said filter means for determining the presence of speech elements having characteristic frequency spectra.

16. Apparatus for real-time speech element identification according to claim 15 and wherein said means for selectively governing is operative to provide detection of a plurality of discrete frequencies in real time using a single filter.

17. Apparatus for real-time speech element identification according to claim 15 and wherein said detector means comprises a plurality of detectors having different detection frequency bands.

18. Apparatus for real-time speech element identification according to claim 15 and wherein said variable delay line means comprises means for selectably feeding electrical signals therethrough a plurality of times.

19. Apparatus according to claim 10 and wherein said gain control means is operative to maintain a stable gain during the presence of speech from a single source.

20. Apparatus according to any of claims 1 - 14 and wherein said transducer means comprises vibration generating means.

0144774

FIG. 1

FIG. 2a

13

FIG. 2b

19

FIG. 2C

26

FIG. 2d

27    27

28    28

FIG. 2e

43

400 HZ

FIG. 2f

45

150 HZ

FIG. 3

0144774

3/6

FIG. 4

FIG. 5

0144774

FIG. 6